# EUROPEAN PATENT APPLICATION

(11) **EP 4 563 153 A1**
(43) Date of publication of application: **04.06.2025**
(21) Application number: 24216673.4
(22) Date of filing: 29.11.2024
(51) Int. Cl.: A61K 31/785, A61K 31/787, A61K 9/28, A61K 9/50, A61P 1/00, A61P 1/16, A61P 3/04, A61P 3/06, A61P 3/10

(54) **METHOD FOR TREATING METABOLIC DISORDERS BY ILEUM-TARGETED DELIVERY OF ENDOTOXIN SEQUESTRANT**

(30) Priority: 30.11.2023 CN 202311629193; 27.11.2024 CN 202411707833
(71) Applicant: SQ Biopharma Inc., Chengdu Sichuan 610219 (CN)
(72) Inventor: HAN, Yuan-ping, Chengdu, 610219 (CN); LIU, Wei, Chengdu, 610219 (CN); QI, Yucheng, Chengdu, 610219 (CN)
(74) Representative: Scholl, Matthias

(57) **Abstract**

A method of treating a subject having a metabolic disorder, caused or promoted by pathogen-associated molecular patterns (PAMPs) derived from intestinal sources, including: administering to the subject a specially formulated drug that is targeted to deliver a sequestrant into the distal region of small intestine for sequestration of PAMPs including endotoxins or CpG-DNA derived from gut microbes. The sequestrant binds to the PAMPs to form a sequestrant-PAMP complex; and the sequestrant-PAMP complex is eliminated from the digestive tract along with the rest of digestive contents.

## Description

The disclosure is directed to a method, composition, and formulation of a class of drug, comprising high-molecular-weight sequestrant as active pharmaceutical ingredient, which is targeted to the distal region of the small intestine. This targeting formulation of the copolymeric sequestrant enables the effective sequestration and excretion of endotoxin and other pathogen-associated molecular patterns. In this way, the drug can be used to suppress the entry of endotoxin into the liver and circulation system for prevention and treatment of metabolic diseases.

Metabolic disorders, in the form of obesity, fatty liver diseases, and type 2 diabetes, among others, have imposed great health challenges and an enormous economic burden on societies worldwide. Studies have shown that metabolic diseases affect a significant proportion of the adult population. According to large-scale surveys, the prevalence ranges from approximately 10% to over 30% depending on the region and population studied. In the United States, metabolic syndrome is a significant public health problem. The high prevalence is attributed at least in part to factors such as a diet rich in processed foods, high fat, high sugar intake, and a relatively sedentary lifestyle. Additionally, the high rates of obesity in the US contribute significantly to the development of metabolic syndrome and diabetes.

Alcoholic liver diseases (ALD) cover a variety of liver disorders. Epidemiological investigations estimate that there are 60 million people in China suffering from alcoholic liver diseases in varying degrees. Severe alcohol abuse can induce extensive hepatocyte necrosis and even liver failure.

Alcohol drinking is associated with a wide range of diseases and health problems. First, excessive alcohol consumption leads to the accumulation of fat in the liver. Heavy drinkers may develop into alcoholic hepatitis (AH), featured as jaundice, abdominal pain, and fatigue. Persistent alcoholic hepatitis may progress into hepatic inflammation and cirrhosis, which may further prognosed into liver failure, cancers, and other complications such as bleeding from enlarged blood vessels in the esophagus. Alcohol drinking may also lead to cardiovascular diseases (CVD), arrhythmias (irregular heartbeats), myocardial damage, esophagitis and gastritis, peptic ulcers, pancreatitis and even cancer.

Blood test for indicators of ALD include elevated levels of serum aspartate aminotransferase (AST), alanine aminotransferase (ALT), gamma-glutamyl transpeptidase (GGT), total bilirubin (TBil), prothrombin time (PT), mean corpuscular volume (MCV), and carbohydrate-deficient transferrin (CDT).

In normal physiological condition, intestinal microbes in massive amount ranging from 1-2 kilograms are mostly restrained in the large intestine, while small intestine in the upper section is near sterile, and such gating mechanism is mediated in part by alpha-defensin 5/6 secreted by Paneth cells located in the crypts of microvilli. Loss function of Paneth cells with decreased expression of the alpha-defensin is often related to many metabolic disorders. Moreover, intestinal impairment, which is often related to aging, and loss of tight junction of intestinal epithelial cells for leaky gut, is also common in the subjects in metabolic disorders.

Vitamin D signaling controls Paneth cell functions in part through promoting expression of alpha-defensin, while vitamin D deficiency, which is commonly associated with metabolic disorders, can lead to down-regulation of the defensin, and small intestinal bacteria over-growth (SIGO), which consequently may promote endotoxemia for insulin resistance, metabolic disorders, and hepatic steatosis.

Pathogen-associated molecular patterns (PAMPs), derived from gut microbes, once entry the body, can promote metabolic disorders through several mechanisms. For instance, PAMPs can activate the innate immune through pattern recognition receptors (such as Toll-like receptors) on immune cells. The interaction of the ligands with the receptors triggers a cascade of signaling events that may lead to the release of pro-inflammatory cytokines such as tumor necrosis factor-alpha (TNF-α), interleukin-6 (IL-6), and interleukin-1β (IL-1β).

Biochemically, endotoxin is lipopolysaccharide (LPS), derived from the cell walls of Gram-negative bacteria. LPS shed from the cell walls of dead bacteria and living organisms contributes to the elevated level of circulating endotoxin, called metabolic endotoxin. LPS levels in the peripheral circulation usually increase after food intake. LPS is incorporated into the chylomicrons and passes across the intestinal barriers into the lymphatic system and then into the bloodstream. In the bloodstream, endotoxin is transported by the LPS-binding protein, called LBP, and others such as HDL-cholesterol.

In physiological condition of health subjects, the endotoxin in complex with HDL-c or LBP is transported to the liver, where it undergoes degradation by specific liver enzymes (such as acyloxyacyl hydroxylase and alkaline phosphatase) or excretion into the bile via scavenger receptors. Kupffer cells in the liver can detoxify endotoxins through phagocytosis. However, with increase of age or various forms of liver injury, the liver cells were unable to clean up the circulating endotoxin, and the consequent endotoxin can spill over into the systemic circulation, resulting in low-grade endotoxaemia, which can further promote insulin resistance for T2D and non-alcoholic fatty liver disease (NAFLD). The pro-inflammatory signaling exerted by LPS occurs via binding of its lipid moiety lipid A to TLR4 in combination with the membrane-bound co-receptor CD14. LPS binding to TLR4 leads to the recruitment of the adaptor protein, MyD88, to the cytoplasmic domain of TLR4, resulting in activation of the transcription factor NF-κB among others for transcriptional cascade and inflammatory reaction.

Endotoxin in the small intestine can also be absorbed via portal vein into the liver, which is particularly true in the situation of aging and impaired intestine in variety of reason. Endotoxemia is generally considered to be a state where the plasma endotoxin level rises to greater than 2.5 EU (endotoxin units)/mL or 0.25-0.5 pg/mL.

Compelling clinical studies have found strong association of endotoxemia with a variety of diseases, especially liver diseases. Long-term alcohol consumption, aging, viral infection, and vitamin D deficiency, may lead to impaired integrity for leaky gut. In those situations, bacteria in the large intestine can migrate into the small intestine, called small intestinal bacterial overgrowth (SIBO), which contribute the enrichment of endotoxin among other PAMPs in the distal region of small intestine.

A large number of studies have shown that the plasma endotoxin level in patients with alcoholic liver diseases is several times that of healthy subjects. The plasma endotoxin level in normal subjects is between 0.3-10.4 pg/mL, while that in patients with alcoholic liver diseases is between 8.5-206 pg/mL. Although endotoxin has a wide variation among different individuals, the plasma endotoxin level in the patients with alcoholic liver diseases is always 5-20 times that of normal subjects. Animal experimental models of alcoholic liver injury have confirmed the endotoxemia in alcoholic liver diseases. Based on male rat model and through alcohol binge, one report showed that acute or chronic ethanol administration increases the plasma endotoxin level, and this level is closely related to the development of liver injury. Another work based on ethanol-fed rats showed that increased level of endotoxemia and lipid peroxidation in females is associated with NF-kappa B activation and chemokine production, leading to liver injury. The alleviation of alcohol-induced endotoxemia and liver injury by antibiotics indicates that endotoxin plays a crucial and causal role in alcoholic liver injury. Indeed, oral administration of large amount of polymyxin B, through sterilization of gut microbes and depletion endotoxin can improve alcohol induced hepatitis in Wistar rat. Emerging evidence shows that LPS and ethanol synergistically affect liver cells. LPS alone cannot mimic ethanol-induced steatosis or hepatitis, but ethanol and LPS together effectively induce liver injury. Ethanol feeding makes the liver of experimental animals sensitive to LPS-induced cellular damage.

In the circulation system, LPS-binding protein (LBP) presents the bacterial toxin to the cellular receptors CD14 and TLR-4. Consequently, chronic ethanol feeding enhances the expression of LBP and CD14, and conversely, the gene knockout mice of LBP, CD14, and TLR-4 exhibited resistance to ethanol challenge, showing causative role of endotoxin in promoting of liver injury.

Likewise, reduction of endotoxins from the bloodstream has been demonstrated as an effective method to treat liver diseases in severe conditions. For such regard, polymyxin B immobilized fiber columns (PMX) in the device of extracorporeal circulation have been used clinically for removal of endotoxin in liver failure and septic shock. The extracorporeal circulation or plasma exchange has been used to reduce hepatic toxins and endotoxins for treatment of various diseases such as liver failure, multiple organ failure, and sepsis. However, high cost and surgical risks are significant obstacles for the extracorporeal-circulation methods including PMX. Importantly, these treatments are unable to block the influx of the intestinal source of endotoxin into the body. Thus, development of oral administered drug that can sequestrate intestinal PAMP including endotoxin will have profound potential application for prevention and treatment of various metabolic disorders including the liver diseases.

Bile acid sequestrants, including cholestyramine, colesevelam, colestipol, are biocompatible resins that are not absorbed and can be totally excreted from the gastrointestinal tract after binding bile acids. These types of high molecular weight copolymers have been used in clinical treatment for reduction of hypercholesterolemia through sequestering bile acids, by which cholesterol is converted to bile acid in a compensatory way.

In the present disclosure, specific polymeric sequestrants can be utilized as active pharmaceutical ingredients for sequestrating the intestinal sources of endotoxin, as well as other pathogen-associated molecular patterns (PAMPs). Moreover, in this disclosure we present a targeted delivery system to send the abovementioned sequestrants to the distal region of small intestine to achieve high efficiency of sequestration and therapeutic potential. This disclosure improves not only the efficiency of sequestration of endotoxin and other PAMs richen in the ileum, but also the safety of the therapeutics through reduced dosage and limited adsorption of hydrophobic nutrients in major section of small intestine. These two elements thus constitute the foundation of novel therapy, named as PASA (Pathogen Adsorption and Sequestration Agents) therapy. In current application, a method is disclosed for preparation of ileum-targeted deliver of endotoxin sequestrant and related therapeutic applications.

The disclosure relates to a method for drug preparation and the potential therapeutic application. Specifically, the method involves using specific copolymers as the active pharmaceutical ingredient and formulating the drug for targeted delivery to the ileum, which can effectively sequestrate endotoxins and other PAMPs for therapeutic application. The method offers distinct advantages in the field of pharmaceuticals, enabling precise and effective drug delivery for sequestrating endotoxins richen in the distal region of small intestine. The method has significant potential for the treatment of various medical conditions and represents a novel and valuable contribution to the art of drug preparation and therapeutic application.

In one aspect, the disclosure provides a sequestrant for use in the treatment of a subject having a metabolic disorder, caused or promoted by endotoxins or other PAMPs derived from intestinal source. The sequestrant comprises at least one biocompatible material (a particle, a polymer, or a combination thereof) as active pharmaceutical ingredient (API). The API is non-digestible and non-absorbable in the digestive tract of the subject. The sequestrant specifically binds to PAMPs, such as endotoxins and CpG-DNA. CpG-DNA is derived from gut microbes and triggers immune activation and inflammation. When the sequestrant binds to PAMPs in the digestive tract, a sequestrant-PAMP complex is formed. The binding involves covalent or noncovalent bonds, allowing the sequestrant-PAMP complex to remain intact within the gut. The sequestrant-PAMP complex is eventually excreted from the digestive tract along with the rest of digestive contents.

The sequestrant is formulated in the form of a pellet, tablet, or capsule containing API for sequestration of PAMPs. The pellet, tablet, or capsule comprises a core granulate, an isolation layer, and a pH-sensitive coating layer. The core granulate comprises a copolymer as API. The copolymer is insoluble and indigestible during digestion and released in the distal region of small intestine to bind with PAMPs.

In another aspect, the disclosure provides a method of treating a subject having a metabolic disorder, caused by persistent inflammation and the consequent insulin resistance. The metabolic disorder is, in part, driven by endotoxins. The metabolic disorder comprises alcoholic hepatitis, metabolic associated steatohepatitis (MASH), liver fibrosis and cirrhosis, liver failure, and liver cancer. The metabolic disorder also promotes cardiovascular diseases (CVD), type-2 disease (T2D), and central obesity.

The following advantage are associated with the disclosure:
The existing therapeutic treatments for metabolic diseases such as nonalcoholic steatohepatitis (NASH), metabolic-associated steatohepatitis (MASH), and alcoholic hepatitis are mainly focused on targeting specific cellular components, such as key enzymes and receptors involved in the metabolic pathways. For example, obeticholic acid (OCA), a new drug in development for NASH, functions as a farnesoid X receptor (FXR) agonist. FXR is a nuclear receptor that has a crucial role in the regulation of bile acid, lipid, and glucose metabolism. By activating FXR, OCA improves liver function and reduces inflammation and fibrosis in NASH patients. Although OCA has shown some benefits in NASH patients, the magnitude of improvement may not be sufficient to meet the regulatory requirements for approval. Elafibranor, a dual peroxisome proliferator-activated receptor (PPAR) alpha/delta agonist, aims to improve insulin sensitivity, lipid metabolism, and reduce inflammation. Unfortunately, elafibranor fails in clinical trials, underscoring the difficulty of achieving reliable results in such a complex disease. The pathogenesis of NASH is highly variable across individuals, as genetics and environmental factors influence how patients respond to treatments. The variability is partly due to hundreds of genes and single nucleotide polymoepgisms (SNPs) associated with the risk NASH. For instance, genes involved in lipid metabolism, such as PNPLA3 (patatin-like phospholipase domain-containing protein 3), TM6SF2 (transmembrane 6 superfamily member 2), and MBOAT7 (membrane-bound O-acyltransferase domain-containing 7), have been strongly associated with an increased risk of NASH. These genes play roles in processes like triglyceride metabolism and lipid droplet formation. Moreover, the genes related to inflammation and immune response, such as those encoding cytokines and chemokines, also contribute to NASH pathogenesis. Indeed, genes involved in the tumor necrosis factor-alpha (TNF-α) pathway and interleukin-6 (IL-6) signaling can influence inflammation in the liver. The genes involved in oxidative stress and mitochondrial function, like SOD2 (superoxide dismutase 2), may also be involved in NASH. Mitochondrial dysfunction and related genes can also lead to increased oxidative stress and contribute to liver damage. These small molecular weight drugs that target specific pathways are ineffective in addressing the wide diversity of pathogenesis in NASH. The same is true for other metabolic diseases. Therefore, we need to think creatively and address the upstream sources or causes of liver diseases. In this regard, a novel therapeutic method called PASA (Pathogen Adsorption and Sequestration Agents) therapy is proposed herein. The method shows promise in treating alcoholic hepatitis and other metabolic disorders. By targeted release of copolymer-based sequestrants in the distal region of small intestine, the drug can efficiently adsorb and sequester the microbe-derived pathogens such as endotoxins, and thus block the upstream and intestinal source of inflammation, which provides a potential solution to tackle the underlying causes of metabolic diseases.

Endotoxins and other pathogen-associated molecular patterns (PAMPs) that emerge from bacterial translocation from the colon into the small intestine can enter the circulation and the liver through two main routes: adsorption by the lymphatic system or the portal vein respectively. Such elevated level of metabolic endotoxin from the gut is a major source of systemic and tissue inflammation. For example, metabolic endotoxin can promote insulin resistance by inactivating the signal transduction pathway mediated by insulin. This is the basis for type-2 diabetes and other related metabolic disorders.

Studies indicated that the bile acid sequestrants such as cholestyramine and colesevelam have the ability to efficiently bind and sequester endotoxins and bacterial CpG-DNA. Additionally, oral administration of these sequestrants can reduce metabolic endotoxin levels in other pathological conditions such as metabolic syndrome, liver fibrosis, and pancreatic cancer. However, these high molecular weight sequestrants in their original formulation are designated to release in the upper section of the gastrointestinal tract, which are less efficient to capture endotoxins, mostly richen in the distal region of the small intestine. Additionally, the previous formulation of these sequestrants dispersed in the upper part of the small intestine may also sequester hydrophobic nutrients such as vitamin D and vitamin K. In response to these concerns and drawback, the disclosure presents a novel method for the preparation, composition, and formulation of a kind of pharmaceutical agents through targeting the new pathogens for novel medical application and indications.

In the first part of the disclosure, the method for the preparation of pH-sensitive and time-dependent formulations for targeted release the sequestrants in the distal region of the small intestine are described. The methods can overcome the defects of existing methods and drugs, showing efficient sequestration of endotoxins in the distal region of small intestine, and minimization of the binding of hydrophobic nutrients as well.

In the second part of this disclosure, the ileum-targeted release of the sequestrants can be utilized as a therapeutic approach for the treatment of related metabolic disorders such as alcoholic hepatitis. The medication, the ileum-targeted colesevelam or cholestyramine, can suppress systemic inflammation, and hepatic inflammation, the prominent factors for many diseases. These findings thus constitute the basis for expanding the indications to attenuate other liver diseases.

At molecular levels, the ileum-targeted sequestrants can modulate many pathological pathways including (1) hepatocellular carcinoma, (2) diabetic complications, (3) p53, (4) Hippo, (5) insulin resistance, (6) cellular senescence, (7) rheumatoid arthritis, (8) drug metabolism, (9) chemokine signaling, (10) Th1 versus Th2 cell differentiation. This global modulation of hepatic pathways also supports the basis of PASA therapy for its wide application as the novel therapy.
FIG. 1: A photography image shows the prototypes of pH-sensitive and ileum-targeted release of co-polymers. In this context, SQ1-T represents ileum-targeted cholestyramine, and SQ1-C stands for ileum-targeted colesevelam. Meanwhile, the ileum-targeted barium sulfate is utilized for X-ray based tracking to determine the positioning and timing of the intestinal release.
FIG. 2: Computed tomography based X-ray photography was used to determine the disintegration time and position for the ileum-targeting release of endotoxin sequestrants, SQ1-T for ileum-targeting release of cholestyramine, and SQ1-C for ileum-targeting release of colesevelam.
FIG. 3: The pH6.5-disintegrated SQ1 formulation of ileum-targeted release of cholestyramine has the capacity for *in vitro* sequestration of LPS (lipopolysaccharide) and CpG-DNA fragments.
FIG. 4A: Two prototypes of pH-sensitive-and-ileum-targeted pellets. SQ6.5 for pH6.5-disintegrated colesevelam, and SQ7.5 for pH7.5-released colesevelam.
FIG. 4B: The *in vitro* disintegration time and pH by the pH-sensitive-and-ileum-targeted mini tablets. SQ6.5 for pH6.5-disintegrated colesevelam, and SQ7.5 for pH7.5-released colesevelam.
FIG. 5: *In vitro* disintegration of a formulation of pellets, namely barium sulfate formulations with pH dissolving values at 6.5, was recorded at 37°C rocking for 6 hours in the buffer solution. The buffer solutions were (a) 0.1 HCl, (b) pH6.5 buffer with 50 mM Tris and 120 mM NaCl, and (c) pH7.5 buffer with 50 mM Tris and 120 mM NaCl.
FIG. 6: The *in vivo* disintegration of two formulations of pellets, namely barium sulfate formulations with pH dissolving values at 6.5 and 7.5 respectively, was recorded by X-ray photography of Sprague Dawley rats at the specified time points.
FIG. 7: Experiment design #1. Acute alcoholic hepatitis in young rats and the therapeutic efficacy of pH-6.5 ileum-targeted colesevelam and API powder suspension. Seven-week-old Sprague Dawley rats were primed with alcohol for six weeks using the Lieber-DeCarli diet containing 5% alcohol. Then, the rats were subjected to three consecutive binges at 5g/kg body weight. From the fourth week on, SQ1-C (the pH-6.5 ileum-targeted formulation of colesevelam) and the respective suspension powder were administered orally at 91 mg/kg body weight.
FIG. 8: Plasma endotoxin levels in young Sprague Dawley rats were increased in alcohol-induced liver injury, and suppressed through treatment with pH 6.5-and-ileum-targeted colesevelam (SQ1-C) and the respective colesevelam suspension (CV). The experimental design was described in FIG. 7. The plasma endotoxin levels were measured by the LAL (Limulus Amebocyte Lysate) assay.
FIG. 9: Systemic inflammation induced by alcohol and its suppression by oral administration of ileum-targeted SQ1-C and CV was determined through measurement of the serum levels of interleukin-1β. The experimental design was described in FIG. 7. The cytokine was measured by the ELISA assay.
FIG. 10: Oral administration of SQ1-C, the pH 6.5-and-ileum-targeted colesevelam, and its corresponding active pharmaceutical ingredient (API) named as CV, can suppress the alcohol-induced systemic inflammation as measured by the serum levels of interleukin-6. The experimental design was described in FIG. 7. The cytokine was measured by the ELISA assay.
FIG. 11A: The scoring scale of hepatic steatosis was determined through Hematoxylin and Eosin staining of the liver tissues from the aforementioned alcoholic hepatitis. The experimental design was described in FIG. 7.
FIG. 11B: Alcohol treatment leads to increased hepatic steatosis, while oral administration of SQ1-C, an ileum-targeted formulation of colesevelam, can alleviate the steatosis.
FIG. 12A: The standard, criteria of index, and scoring of hepatic inflammation.
FIG. 12B: The inflammatory index in the livers of rats that have consumed alcohol for 6 weeks and then undergone three binge - drinking treatments is elevated. However, this increase is alleviated by SQ1-C, the ileum-targeted formulation of colesevelam.
FIG. 13A: Increased hepatic inflammation, as determined by the mRNA level of interleukin-1 β (panel A) in the rats was alleviated by administration of SQ1-C, ileum-targeted formulation of colesevelam at a dosage at 91 mg/kg daily.
FIG. 13B: Increased hepatic inflammation, as determined by the mRNA level of interleukin-6 (panel B) in the rats was alleviated by administration of SQ1-C, ileum-targeted formulation of colesevelam at a dosage at 91 mg/kg daily.

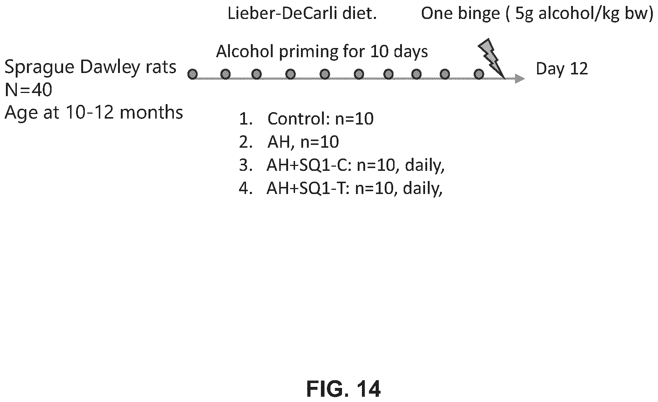

FIG. 14: An experimental design #2. Acute alcoholic hepatitis in middle-aged rats and a therapeutic efficacy test of pH-sensitive and ileum-targeted colesevelam and cholestyramine. Forty middle-aged rats (aged 10-12 months) were utilized. Thirty of them were primed with alcohol in a Lieber-DeCarli diet for ten days, followed by a binge treatment at 5g/kg body weight. The rats were terminated within 24 hours. During the experiment course, two types of mini tablets, SQ1-C and SQ1-T, which are pH 6.5-ileum targeted pellets containing colesevelam and cholestyramine respectively, were orally administered at a dose of 91 mg/kg daily (n=10 for each group).
FIG. 15: The pH-sensitive and ileum-targeted endotoxin sequestrants, colesevelam and cholestyramine, possess the ability to suppress the elevated levels of plasma endotoxin in acute alcoholic hepatitis. As depicted in FIG. 14, in middle-aged rats being subjected to alcohol priming in combination with one binge treatment led to an increase in plasma endotoxin levels, referred to as metabolic endotoxin. Notably, SQ1-C and SQ1-T can statistically suppress the plasma levels of endotoxin. The plasma endotoxin levels were measured by the LAL (Limulus Amebocyte Lysate) assay.
FIG. 16A: Through intestinal sequestration, the pH-sensitive and ileum-targeted endotoxin sequestrants are able to suppress serum levels of TNF-alpha in acute alcoholic hepatitis, as described in FIG. 14.
FIG. 16B: The pH-sensitive and ileum-targeted endotoxin sequestrants are able to suppress serum levels of interleukin-1 beta in acute alcoholic hepatitis.
FIG. 17A: Oral administration of the pH-sensitive and ileum-targeted endotoxin sequestrants can suppress the surged levels of alanine aminotransferase (ALT) in the middle-aged rats under acute alcoholic hepatitis, as described in FIG.14.
FIG. 17B: Oral administration of the pH-sensitive and ileum-targeted endotoxin sequestrants can suppress the surged levels of aspartate aminotransferase (AST) in the middle-aged rats under acute alcoholic hepatitis, as described in FIG 14.
FIG. 18A: The increased prothrombin time (PT) in alcoholic hepatitis, is suppressed by oral administration of SQ1-C and SQ1-T in the rat model as mentioned in FIG. 14, being determined by Automated Chemistry Analyzers.
FIG. 18B: The activated partial thromboplastin time (APTT) in alcoholic hepatitis, is suppressed by oral administration of SQ1-C and SQ1-T in the rat model as mentioned in FIG. 14, being determined by Automated Chemistry Analyzers.
FIG. 19A: Impairment of pancreatic function in alcoholic treatment, as measured by the increased level of a plasma pancreatic marker, alkaline phosphatase (ALP), can be inhibited by oral administration of SQ1-C and SQ1-T in the rat model as mentioned in FIG. 14, being determined by Automated Chemistry Analyzers.
FIG. 19B: Impairment of pancreatic function in alcoholic hepatitis, as measured by the increased level of a plasma pancreatic marker, alpha-amylase (P-AMY), can be inhibited by oral administration of SQ1-C and SQ1-T in the rat model as mentioned in FIG. 14, being determined by Automated Chemistry Analyzers.
FIG. 20: Elevated glucose level or hyperglycemia in alcoholic hepatitis, is attenuated by oral administration of SQ1-C and SQ1-T in the rat model as mentioned in FIG. 14 in the plasma levels of triglyceride and glucose, being determined by Automated Chemistry Analyzers.
FIG. 21A: The GO analysis for the hepatic gene expression by the middle-aged rats under acute alcoholic hepatitis versus the control. The GO (Gene Ontology) enrichment analysis provides insights into the biological processes that are differentially regulated between the model group (the SD rats in alcoholic hepatitis) versus the control.
FIG. 21B: The KEGG analysis for the hepatic gene expression by the middle-aged rats under acute alcoholic hepatitis versus the control. The KEGG enrichment analysis results for alcoholic model vs control provide insights into the differences between animals with alcohol binge and the control group in rats, in the experiment mentioned in FIG. 14.
FIG. 22A: The KEGG enrichment analysis for the hepatic gene expression by the middle-aged rats under acute alcoholic hepatitis vs treatment with ileum-targeted endotoxin sequestrants, SQ1-C.
FIG. 22B: Another KEGG enrichment analysis for the hepatic gene expression by the middle-aged rats under acute alcoholic hepatitis vs treatment with ileum-targeted endotoxin sequestrants, SQ1-T.
FIG. 23A: The elevated hepatic inflammation and pathological progression, showing increased expression of hepatic TNF-alpha receptor family gene (panel A) in mRNA levels, in alcoholic hepatitis, is inhibited by oral administration of SQ1-C and SQ1T in the experiment mentioned in FIG. 14.
FIG. 23B: The elevated hepatic inflammation and pathological progression, showing increased expression of hepatic thrombospondin 1 (panel B) in mRNA levels, in alcoholic hepatitis, is inhibited by oral administration of SQ1-C and SQ1-T in the experiment mentioned in FIG. 14.
FIG. 24A: The hepatic fibrogenesis and pathological progression, showing increased expression of hepatic lysine oxidase 2 (Lox 2, panel A) in mRNA levels, which is a prominent factor for cirrhosis in alcoholic hepatitis, can be inhibited by oral administration of SQ1-C and SQ1-T in the rat model, as mentioned in FIG. 14.
FIG. 24B: The hepatic fibrogenesis and pathological progression, showing increased expression of transforming growth factor beta-2 (Tgfb2, panel B) in mRNA levels, which is a prominent factor for cirrhosis in alcoholic hepatitis, can be inhibited by oral administration of SQ1-C and SQ1-T in the rat model, as mentioned in FIG. 14.

### Example 1

Preparation and formulation of pH-sensitive-and-ileum-targeted mini pellets or tablets containing the sequestrant as API.

In the experiment, selected copolymers, such as cholestyramine, colesevelam, colestipol, and others, were used as the active pharmaceutical ingredient (API) to prepare a pH-sensitive-and-ileum-targeted formulation. The formulation process relied on an Eudragit system for achieving pH sensitivity. Specifically, poly methacrylic acid-co-acrylates in the trade name of Eudragit S100, and poly methacrylic acid-co-ethyl acrylate, in the trade name of Eudragit L100, two commonly used polymethacrylate copolymers, were chosen due to their differing characteristics in drug delivery applications. Eudragit S100 is an anionic polymer composed of methyl methacrylate (MMA) and methacrylic acid (MAA) in a molar ratio of approximately 1:2, with a molecular weight of about 135 kDa. Eudragit S100 dissolves in environments with a pH greater than 7, making it suitable for colon-targeted drug delivery systems, as the pH in the colon is typically above 7. Eudragit L100 is also an anionic polymer, with a molar ratio of MAA to MMA of about 1:1 and a similar molecular weight of approximately 135 kDa. Eudragit L100 is typically a copolymer of methacrylic acid (C₄H₆O₂) and methyl methacrylate (C₅H₈O₂). Methacrylic acid contains a carboxylic acid group (-COOH) attached to a carbon chain with a double bond. Methyl methacrylate, is the methyl ester of methacrylic acid. Eudragit L100 dissolves in environments with a pH ranging between 6 and 7.

### Example 2

A protocol for preparation of pH-6.5 and pH-7.5 targeted colesevelam or cholestyramine for ileum-targeted release using Eudragit system (Table 1, Table 2).

**Table 1. An embodiment of formulation for the core granulates of the tablets of the ileum-targeted release of copolymer-based endotoxin sequestrant**

| Ingredients | grams |
|---|---|
| API, specific cationic copolymers | 70.0 |
| Microcrystalline cellulose | 25 |
| Sodium carboxymethyl cellulose | 4.5 |
| Magnesium stearate | 0.5 |
| Total | 100 |

**Table 2. An embodiment of formulation of the coating layer of the pellets or tablets designated for ileum-targeted release of copolymer-based endotoxin sequestrant**

| | |
|---|---|
| Eudragit L100 containing methacrylic acid and methyl methacrylate copolymer. It is designed to dissolve at a pH of around 6.0-7.0 | 6 g |
| Or Eudragit S100 dissolves in environments with a pH greater than 7. | |
| Triethyl citrate. | 0.6 g |
| Talc. | 3.0 g |
| Alcohol | 85.4 g |
| water | 5.0 g |

(1). Introduction: This protocol was designed to create a colesevelam or cholestyramine formulation that is targeted to release in the ileum at a pH of approximately 6.5-7.5 using the Eudragit system.
(2). Materials and Equipment: (A) Colesevelam or cholestyramine powder as API. (B) Eudragit polymer suitable for pH-dependent release (e.g., Eudragit L100 for pH6.5, and Eudragit S100 for pH7.5 version. (C) Organic solvents (e.g., ethanol, isopropyl alcohol). (D) High-shear mixer. (E) Spray dryer. (F) pH buffer solutions for testing.
(3). Formulation Composition: (A) The active ingredient, colesevelam or cholestyramine, was selected for targeted ileum release. (B) Eudragit Polymer was chosen for remaining insoluble in acidic conditions but dissolving at a pH close to 6.5, to achieve targeted release in the ileum. (C) Optional additives such as plasticizers, stabilizers, or glidants were also included to improve the properties of the formulation.
(4). Preparation Steps: (A) The Eudragit polymer was dissolved in an organic solvent, such as ethanol, isopropyl alcohol, or acetone, to form a polymer solution. (B) Colesevelam powder was added to the polymer solution and mixed thoroughly with a high-shear mixer. (C) The concentration and viscosity of the mixture were adjusted to achieve desired properties for coating. (D) A spray dryer was then used to apply the Eudragit polymer onto the colesevelam or cholestyramine granulates, forming coated particles. (E) Optionally, the coated particles were sieved or milled to produce a uniform particle size distribution. (F) The coated colesevelam formulation was tested in buffer solutions at different pH levels, from acidic to alkaline. The testing confirmed that the release was targeted at pH 6.5.
(5). Characterization and Quality Control: (A) Particle size analysis: The particle size of the coated colesevelam was determined using laser diffraction or microscopy. (B) Encapsulation efficiency: The amount of colesevelam encapsulated within the Eudragit coating was assessed by analytical methods. (C) In vitro release studies: Release studies were conducted in pH buffer solutions simulating the gastrointestinal tract, monitoring the release of colesevelam over time to ensure targeted release at pH 6.5. (D) Stability testing: The formulation was stored under different conditions (e.g., temperature, humidity) to monitor changes in particle size, encapsulation efficiency, and release characteristics over time.
(6) Conclusion: The protocol successfully prepared a colesevelam formulation with targeted release in the ileum at pH 6.5 and pH 7.5 respectively, using the Eudragit system. The formulation has the potential to improve the therapeutic efficacy of endotoxin sequestrant such as colesevelam or cholestyramine by ensuring release at intended site in the gastrointestinal tract.

### Example 3

An alternative protocol for preparation of the pH-sensitive and ileum-targeted colesevelam and cholestyramine mini tablets by Eudragit system (Table 3 and Table 4).

**Table 3. An embodiment of formulation for SQ6.5 pellets or mini tablets, designated for disintegration of colesevelam at pH 6.5**

| Composition | Percent |
|---|---|
| Colesevelam hydrochloride | 58.3% |
| Microcrystalline cellulose | 20.8% |
| Sodium carboxymethyl cellulose | 3.8% |
| magnesium stearate, | 0.4% |
| EUDRAGIT^{®} L 100 | 10.4% |
| triethyl citrate, | 1.0% |
| Talc | 5.2% |
| Total | 100% |

**Table 4. An embodiment of formulation for SQ7.5 pellets or mini tablets, designated for disintegration of colesevelam at pH 7.5**

| Composition | Percent |
|---|---|
| Colesevelam hydrochloride | 58.3% |
| Microcrystalline cellulose | 20.8% |
| Sodium carboxymethyl cellulose | 3.8% |
| magnesium stearate, | 0.4% |
| EUDRAGIT^{®} S 100 | 10.4% |
| triethyl citrate, | 1.0% |
| talc | 5.2% |
| Total | 100% |

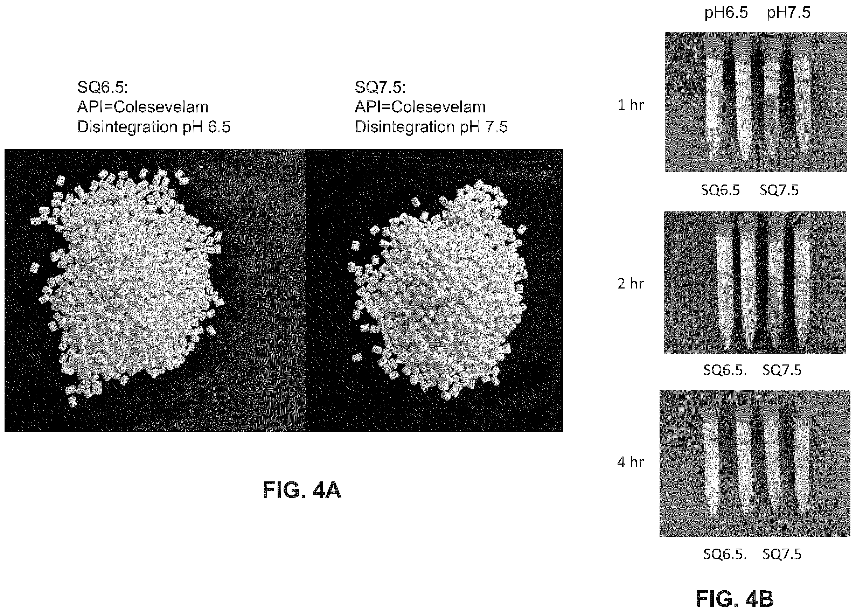

(1) Composition: The core granulates contained bile acid sequestrants such as colesevelam or cholestyramine as API, and other components. The other components included lactose, microcrystalline cellulose, pregelatinized starch, starch, sodium carboxymethyl cellulose, hydroxypropyl methylcellulose, and magnesium stearate. The pH-sensitive enteric layer contained Eudragit S100 (a methacrylic acid-ethyl acrylate copolymer), hydroxypropyl methylcellulose acetate succinate, carboxylated agarose, carboxymethyl yellow lemon calcium chloride hydrogel, anionic copolymers of methacrylic acid and methyl methacrylate, cellulose acetate phthalate, hydroxypropyl methylcellulose phthalate, polyvinyl acetate phthalate, hydroxypropyl methylcellulose acetate succinate, carboxymethyl cellulose, cellulose acetate, and maleic acid and phthalic acid derivative copolymers. The coating layer comprised Eudragit L100 (or Eudragit S100) (5%-7%, w/w), triethyl citrate (0.5%-0.7%), talcum powder (4%-5%), ethyl alcohol (83%-87%), and purified water (4%-6%).
(2) Process: The prescribed amount of colesevelam hydrochloride or cholestyramine was mixed with microcrystalline cellulose and sodium carboxymethyl cellulose in a rotating container set at 15 rpm for 15 minutes to ensure even distribution. Then, 5% hydroxypropyl methylcellulose (w/w) was slowly added, followed by wet granulation and drying in a forced-air drying oven at 60°C. Magnesium stearate was then added, and the mixture was compressed into mini pellets with a 2 mm punch. The core granulates were then coated with an enteric coating containing Eudragit L100 or Eudragit S100, along with other components.
(3) Disintegration time and pH measurement: The pellets were named as SQ6.5 and SQ7.5 to represent their disintegration pH values of 6.5 and 7.5, respectively. As shown in Table 5, both types of the mini pellets resisted disintegration in 0.1 N HCl for up to 4 hours. The SQ6.5 pellets disintegrated in a pH 6.5 solution (50 mM Tris at pH 6.5), while the SQ7.5 pellets remained stable at pH 6.5, but thoroughly disintegrated at pH 7.5 within 30 mins. The hardness of the tablets was also assessed, including for the tablets containing barium sulfate used in animal testing (Table 6). The prototypes of the SQ6.5 and SQ7.5 pellets were shown in FIG. 4A.

**Table 5. Disintegration of the pH-sensitive and ileum-targeted pellets or mini tablets**

| Name API | Disintegration time at pH 2.0 for 4 hrs. | Disintegration time at pH 6.5. | Disintegration time at pH 7.5. |
|---|---|---|---|
| SQ6.5, colesevelam | no | < 30 min. | |
| SQ7.5, colesevelam | no | | < 30 min. |
| Barium sulfate, pH6.5 | no | < 30 min. | |
| Barium sulfate, pH7.5 | no | | < 30 min. |

**Table 6. Hardness of the pH-sensitive and ileum-targeted mini tablets**

| Name, API | Hardness, Newton |
|---|---|
| SQ6.5, colesevelam | 25.04-32.23 |
| SQ7.5, colesevelam | 27.12-33.09 |
| Barium sulfate, pH6.5 | 100.9-112.3 |
| Barium sulfate, pH7.5 | 99.8-110.6 |

### Example 4

T-HPMCP (hydroxypropyl methylcellulose phthalate, Type T) is often used in pH-sensitive formulations for drug delivery. T-HPMCP is a polymer with solubility property that depend on pH. T-HPMCP remains insoluble in acidic environments but becomes soluble at higher pH values. The property makes it ideal for protecting drugs from degradation in the acidic environment of the stomach and ensuring targeted release in the higher pH regions of the intestine.
(1) T-Composition: A T-HPMCP formulation was provided to enable pH-sensitive and ileum-targeted drug release, improving the bioavailability of the API and minimizing side effects. T-HPMCP served as the enteric polymer, controlling the release in the ileum. Additional excipients included fillers (e.g., microcrystalline cellulose), binders (e.g., polyvinylpyrrolidone), lubricants (e.g., magnesium stearate), and disintegrants (e.g., croscarmellose sodium).
(2) Method: Colesevelam, along with the additional excipients, was weighed and blended. Granulation was performed if required. The blend was then compressed into core granulates. A coating of T-HPMCP was applied to the core granulates using pan coating or fluidized bed coating. The coating parameters were adjusted to achieve targeted release of colesevelam in the ileum, with the thickness and composition optimized to keep the formulation intact through the stomach and small intestine, enabling drug release in the ileum.

### Example 5

A protocol for preparation of pH-6.5 targeted formulation for ileum release using a Poly(lactic-co-glycolic acid) (PLGA) system.
(1) Introduction: This protocol was designed to create a formulation that releases a copolymer as API at an ileal pH of 6.5. PLGA was selected as the pH-sensitive polymer due to its ability to degrade and release encapsulated substances in response to specific pH changes.
(2) Materials and Equipment: PLGA with appropriate molecular weight and degradation characteristics; copolymer such as colesevelam and cholestyramine as the API; organic solvents such as dichloromethane or acetone; ultrasonicator; rotary evaporator; lyophilizer; pH buffer solutions for testing.
(3) Formulation Composition: PLGA was served as the polymer to encapsulate with the API, and the molecular weight and copolymer ratios were chosen to ensure controlled degradation and release in the pH range of 6.5-7.5. The API (colesevelam or cholestyramine) was combined with optional additives such as stabilizers, surfactants, or excipients to enhance stability and release.
(4) Preparation Steps: (A) PLGA was dissolved in an organic solvent such as acetone or ethyl acetate to form a polymer solution. The concentration of PLGA was optimized for encapsulation and release. (B) The colesevelam or cholestyramine was added to the polymer solution and mixed thoroughly using an ultrasonicator, ensuring uniform dispersion of the API within the PLGA matrix. (C) An emulsification technique was used to create an emulsion by adding the polymer-API solution into an aqueous phase containing a surfactant. (D) The emulsion was allowed to stabilize, and the organic solvent was removed using a rotary evaporator, resulting in the formation of PLGA nanoparticles encapsulating the API.

### Example 6

A modified version of the pH-sensitive targeted tablet (Table 7).

**Table 7. An embodiment for composition and formulation for the mini pellets of pH 6.8 disintegrated cholestyramine (SQ1-T) or colesevelam (SQ1-C)**

| | Composition | mg | % |
|---|---|---|---|
| Core granule | Colesevelam | 5.6 | 18.86% |
| | Microcrystalline cellulose UF711 | 1.0 | 7.03% |
| | Microcrystalline cellulose 200 | 0.6 | 4.22% |
| | Microcrystalline cellulose KG1000 | 3.4 | 23.89% |
| | Hydroxypropyl methylcellulose | 0.9 | 6.32% |
| | Cross-linked sodium carboxymethylcellulose | 0.2 | 1.41% |
| | Lactose | 2.5 | 17.57% |
| | Magnesium stearate | 0.1 | 0.70% |
| Isolation layer | Methacrylic acid-methyl methacrylate copolymer (1:1) | 0.61 | 4.26% |
| | Triethyl citrate | 0.11 | 0.79% |
| | Absolute ethanol | 8.22 | / |
| Enteric coating | Aqueous dispersion of methacrylic-ethyl acrylate copolymer | 5.84 | 12.30% |
| | Triethyl citrate | 0.28 | 1.97% |
| | talcum powder | 0.27 | 1.87% |
| | Polyethylene glycol 6000 | 0.01 | 0.10% |
| | water | 5.14 | / |

(1). Compression of core granulate. As shown in Table 7, the prescribed amounts of colesevelam or cholestyramine (API), microcrystalline cellulose UF702, microcrystalline cellulose UF711, croscarmellose sodium, and hydroxypropyl methylcellulose were weighed and mixed for 15 minutes to obtain a pre-mixed powder. Magnesium stearate was then added, and the mixture was blended for 5 minutes to form a final powder mixture. The intermediate content was tested, and the tablet weight for compression was calculated based on the intermediate content, with the pellet weight variation controlled within ±5.0%. Using a 2 mm diameter circular deep concave punch, the pellets were compressed, maintaining the hardness within 5-15 N/mm.
(2) Coating with isolation layer. A high-efficiency coating machine was preheated until the exhaust air temperature exceeded 30°C. The prepared core granulates were then placed in the high-efficiency coating machine and random samples were taken for weighing. The atomization and fan pressure were adjusted to 0.1-0.6 MPa, with the inlet and exhaust air temperature maintained at 30±10 °C. The peristaltic pump speed was set to 1-25 revolutions per minute, and the pot body speed to 2-18 revolutions per minute, to achieve a weight gain of 2-3% for the isolation layer.
(3) Pre-preparation of coating. (A) Purified water was added in a stainless-steel bucket. Polyethylene glycol 6000 was dissolved in water with continuously stirring, forming Solution #1. (B) Talc powder was slowly added to another portion of the purified water and stirred for 30 minutes, followed by homogenization at 8000 r/min for 3 minutes, forming a talc powder (Suspension #2). (C) Suspension #2 was cooled. Solution #1 was then added to the cooled with Suspension #2 and stirred for 30 minutes. Triethyl citrate was then added, and the mixture was stirred for 30 minutes, forming Suspension #3. (D) Methacrylic acid-ethyl acrylate copolymer was added in another stainless-steel bucket. The final portion of the purified water was then added, and the mixture was stirred for 30 minutes to ensure uniform dispersion, forming Suspension #4. (E). Suspension #3 was added slowly to Suspension #4, and the mixture was stirred for 60 minutes to form an enteric coating solution with a concentration of 20.0% (w/w). (F) Coating enteric layer. The high-efficiency coating machine was preheated to reach an exhaust air temperature above 30 °C. The core granulate, coated with the isolation layer, was added to the high-efficiency coating machine and preheated for 5 minutes. Random samples were taken for weighing, and the atomization and fan pressure were adjusted to 0.1-0.6 MPa. During the coating process, the inlet and exhaust air temperatures were controlled at 30±10°C. The peristaltic pump operated at 10- 25 revolutions per minute, and the pot rotated at 6-18 revolutions per minute. The weight gain of the enteric layer was controlled to be between 8.0-10.0% (w/w).

The present disclosure describes methods, compositions, and formulations of drugs. The drug formulations comprise sequestrants as the API. These formulations are designed for targeted release in the distal region of the small intestine. The purpose of these formulations is used for sequestering and excreting endotoxins among other PAMP. Additionally, these formulations suppress the entry of endotoxins into the circulation for the medical prevention and treatment of metabolic disorders including liver diseases.

### Example 7

Disintegration *in vivo* of the ileum-targeted colesevelam and cholestyramine by an animal model.

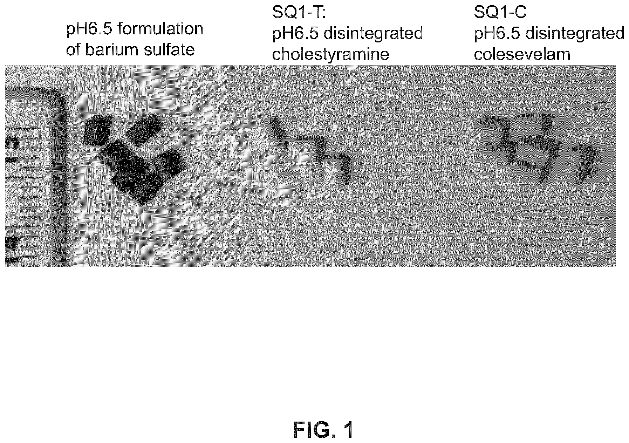

As depicted in FIG. 1, three types of pH-sensitive formulations were prepared as prototypes in pellets. The blue pellets contained barium sulfate for *in vivo* are designated for dissolution at pH 6.8. The white pellets, SQ1-C, were formulated with colesevelam, and the red pellets, SQ1-T, with cholestyramine, both are designed to disintegrate at pH 6.5. The pellets, used in rat experiments, had a diameter of 2 millimeters, a length of 5 millimeters, a hardness ranging from 5 to 15 newtons per square millimeter, and weighed approximately 8-10 milligrams per tablet, containing 5-7 mg of API.

The disintegration pH of the pellets was evaluated by exposing the three types of mini tablets to 0.2 N hydrogen chloride, where all three types of pellets showed resistance for over 60 minutes. Subsequently, the pellets were transferred to buffers with increasing pH values ranging from 2.5 to 7.5, containing 120 mM NaCl and 50 mM Tris. The dissolution time was recorded, and it was observed that the pellets collapsed in a pH 6.5 buffer within 30 minutes. However, the actual dissolution timing *in vivo* was determined by tracing the barium sulfate tablet in animal experiments.

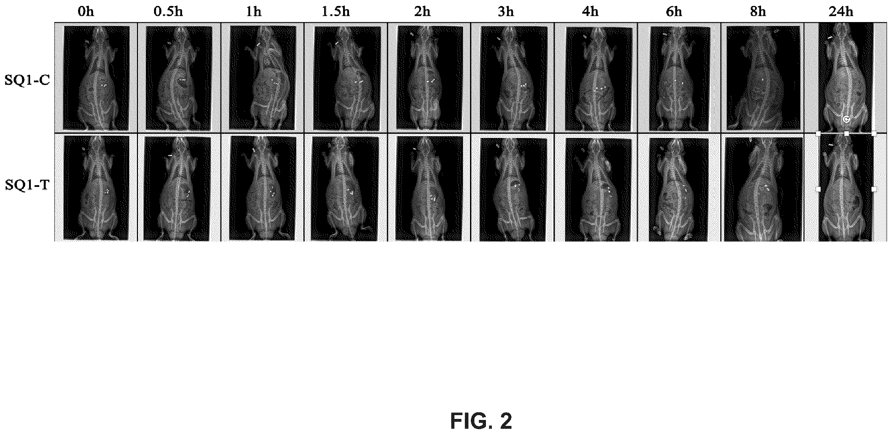

In the *in vivo* disintegration study, adult SD rats were fasted overnight and then orally administered with 4-5 pellets of containing barium sulfate via gavage. Standard chow and drinking water were then provided. Under anesthesia, X-ray CT scans were taken at designated time points to monitor the location and dissolution progress of the pH 6.8 micro-tablet containing barium sulfate. As shown in FIG. 2, the pH 6.8 pellets containing barium sulfate began dissolved between 4 and 6 hours, mostly collapsed by 8 hours, and fully cleared from the gastrointestinal tract within 24 hours post-administration. To verify that disintegration occurred in the distal region of small intestine, additional rats were euthanized and dissected at specific time points to confirm the location of the tablets within the gastrointestinal tract.

### Example 8

*In vitro* sequestration of endotoxin and CpG-DNA.

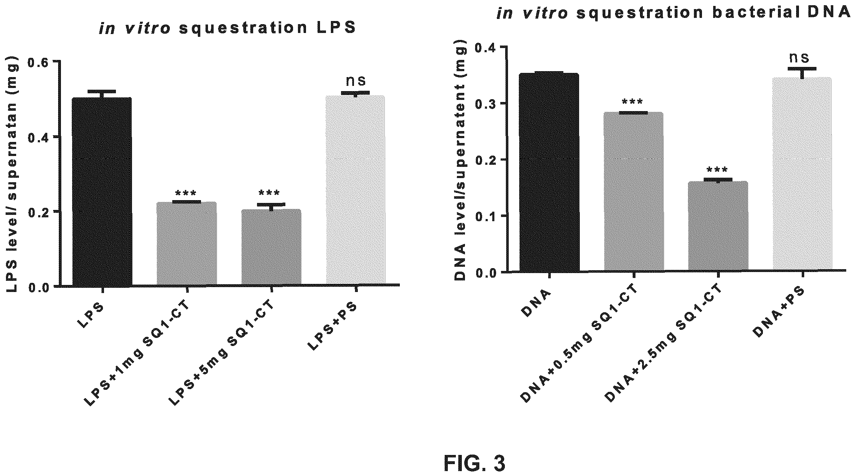

In the animal experiment, endotoxin or lipopolysaccharide in solution was measured using the Limulus Amebocyte Lysate (LAL) assay, which detects the activity of endotoxin. But for in vitro experiment, the pure LPS can also be measured by UV spectrophotometry. For this case, purified endotoxin, with a specific adsorption peak at 258 nm, was used in an *in vitro* binding assay to analyze adsorption and sequestration. As depicted in FIG. 3, the *in vitro* adsorption/neutralization capacity of SQ1-CT along with the API powder suspension was determined. Specifically, LPS (B4, Sigma-Aldrich) was dissolved in pure water, and a specific adsorption peak was scanned and recorded at 258 nm. SQ1-CT and API powder were then dissolved in a pH 6.5 buffer (120 mM NaCl, 50 mM Tris at pH 6.5). The API was collected by centrifugation and the pellets were washed twice with the pH 6.5 buffer, followed by additional centrifugation and collection. Then, a measured amount of LPS was mixed with a measured volume of resin or control buffer. The mixture was incubated for 1 hour and then centrifuged again. The supernatant was collected for spectrophotometry analysis. The results indicated that the SQ1-CT formulation and the API copolymer had similar capacities for adsorbing LPS at pH 6.8. Specifically, 1 gram of SQ1 or colesevelam could adsorb or sequester approximately 0.1 gram of endotoxin *in vitro.* Similarly, bacterial genomic DNA was isolated and purified according to standard procedure, and sequestration capacity was determined as shown in FIG. 3.

### Example 9

Disintegration of two formulations of pH-sensitive colesevelam pellets.

In the experiment, the Eudragit system was used to prepare two types of colesevelam in the form of pellets, named SQ6.5 and SQ7.5, designed to disintegrate at pH 6.5 and pH 7.5, respectively. Each colesevelam pellet contained 4.8 - 5.6 mg of API, with a total weight of about 10 mg (FIGS. 4A-4B).

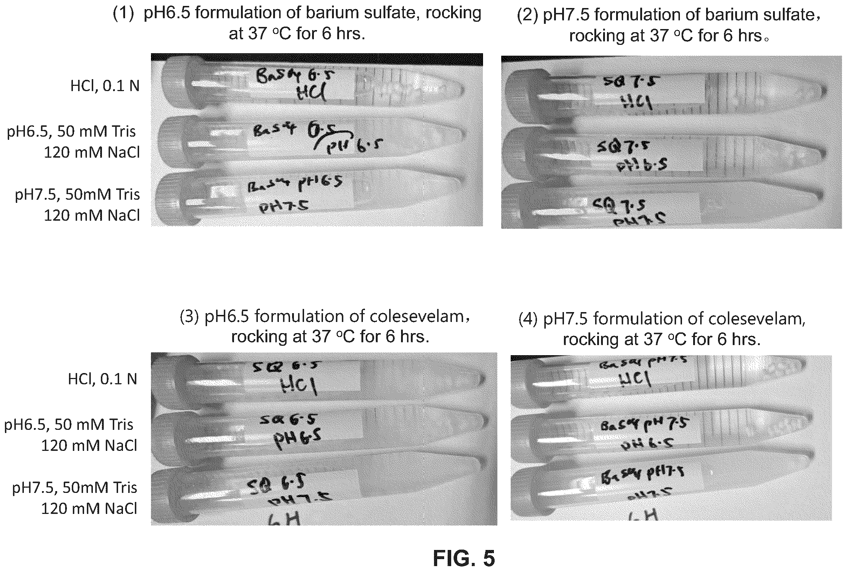

First, the disintegration pH of the mini tablets was measured *in vitro* by placing the pellets in a buffer and rocking them at 37°C. The results showed that both types of the pellets remained stable in 0.1 N hydrogen chloride for up to 4 hours. The pellets were then transferred to buffers with pH values of 6.5 and 7.5, containing 120 mM NaCl and 50 mM Tris, and the dissolution time was recorded. As shown in FIG. 5, the SQ6.5 pellets and a comparable barium chloride pellets were dissolved in the pH 6.5 buffer within 2 hours, while the SQ7.5 formulation and barium chloride remained intact at pH 6.5, but dissolved fully at pH 7.5 within 4 hours.

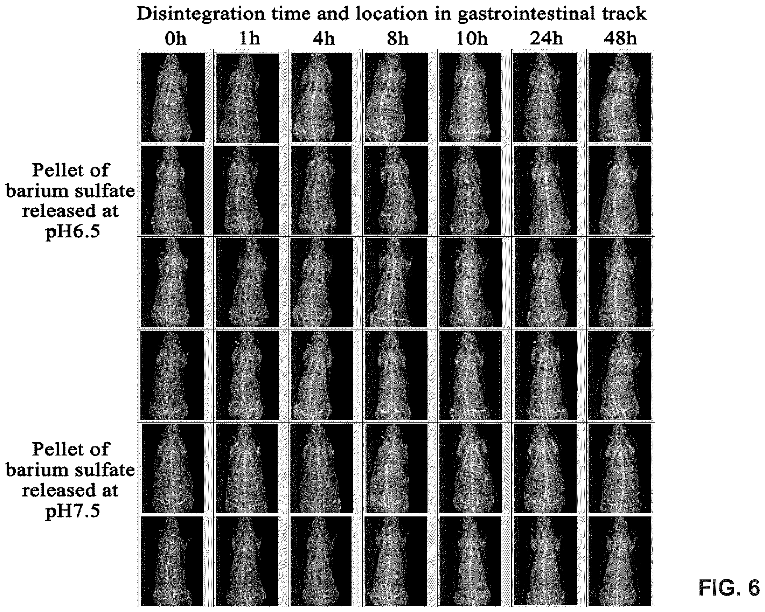

For *in vivo* disintegration studies, adult SD rats were fasted overnight and then orally administered with 5 pellets of the SQ6.5 and SQ7.5 formulation containing barium sulfate via gavage. Then, standard chow and drinking water were then provided. Under anesthesia, X-ray based CT scans were taken at designated time points to monitor the location and dissolution progress of the SQ6.5 and SQ7.5 pellets containing barium sulfate. As shown in FIG. 6, the pH-sensitive pellets began dissolving between 8-10 hours, presumably at the distal region of small intestine. To verify that disintegration occurred in the distal region of small intestine, additional rats were euthanized and dissected at specific time points to confirm the location of the formulation within the gastrointestinal tract.

### Example 10

Experimental design for young rats that undergo three weeks of alcohol priming followed by three binge treatments.

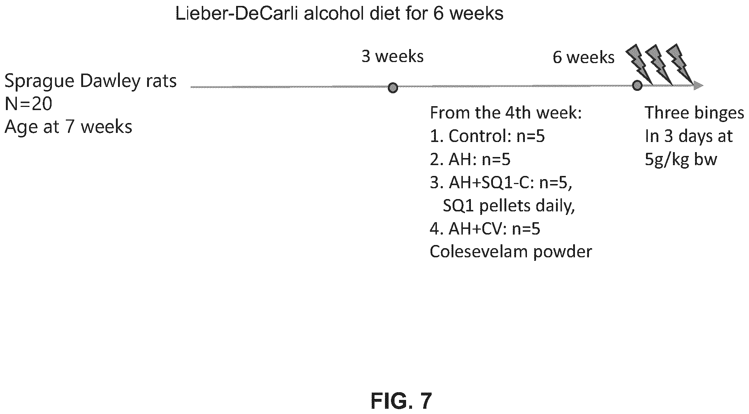

In the experiment, young Sprague Dawley rats were subjected to acute intragastric ethanol feeding (5 g/kg), resulting in rapid increase in endotoxin levels in portal blood from 0 at 60 min to 10 pg/mL at 90 min (Mathurin et al., 2000). Here we modified the method to determine the endotoxin levels in rats subjected to alcoholic hepatitis for three weeks and assess treatment efficacy. As depicted in FIG. 7, twenty 7-week-old SD rats at SPF grade were utilized. Five rats were assigned to a normal control group, while the remaining fifteen rats were fed the Lieber-DeCarli standard alcoholic liquid (containing 36% of total calories from alcohol.) for three weeks to establish an alcoholic hepatitis model. Biochemical tests confirmed liver injury in the rats, as blood transaminase (ALT) levels rising from 25 +/- 10 U/mL in the normal control group to 60 +/- 8 U/mL. The fifteen alcohol-primed rats were then divided into three groups with five rats in each group: one group continued on the alcoholic diet as the model group (AH), the second group was treated with SQ1-C (7 pellets per day, totaling 25 mg API daily via gavage), and the third group was treated with colesevelam powder (CV) at 25 mg daily, also by gavage. These rats were continuously given alcohol diet for additional three weeks, and received a binge treatment with 5 g alcohol per kg of body weight (10 mL per kg). Twenty-four hours after the last binge, the rats were euthanized, and blood, liver, and pancreatic tissues were collected for analysis.

### Example 11

The ileum-targeted colesevelam attenuates the increased levels of metabolic endotoxin in young rats with alcoholic hepatitis.

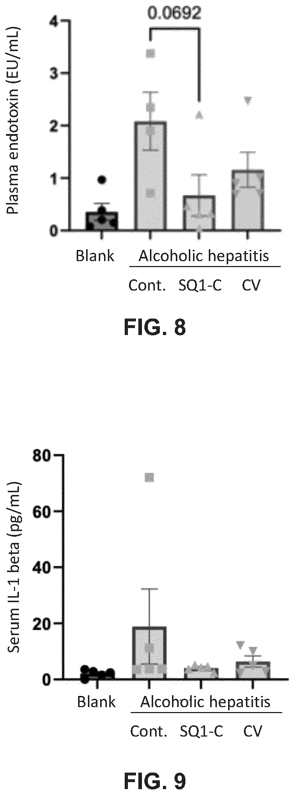

In the experiment, as shown in FIG. 8, the blood endotoxin levels were significantly increased in the young rats subjected to 6 weeks of alcohol priming followed by three binges, as described in FIG. 7. In control rats, the blood endotoxin levels were around 0.3 EU/mL, whereas rats with induced alcoholic liver disease exhibited levels as high as 2.0 EU/mL, indicating a sixfold increase. Administration of SQ1-C pellets reduced the blood endotoxin levels by approximately 75%. In comparison, the colesevelam powder (CV) can partially reduce the blood endotoxin levels.

### Example 12

The ileum-targeted colesevelam ameliorates systemic inflammation in young rat models of alcoholic hepatitis.

Bacterial endotoxins enter the bloodstream and induce the expression of inflammatory factors such as TNF-alpha and IL-1 by activating TLR-4/CD14 cell membrane receptors and associated signaling pathways. Alcoholic liver disease (ALD) is characterized by steatosis and the upregulation of pro-inflammatory cytokines, including IL-1β. IL-1β, type I IL-1 receptor (IL-1R1), and IL-1 receptor antagonist (IL-1Ra) are important regulatory factors in the IL-1 signaling complex that play a role in inflammation. Since IL-1 is significantly associated with acute alcoholic hepatitis symptoms (such as fever, neutrophilia, and cachexia), targeting the IL-1 pathway has potential therapeutic value. The important role of IL-1 type I cytokines and certain inflammasomes has also been confirmed in mouse models of non-alcoholic fatty liver disease. In the young rats subjected to six weeks of alcohol priming followed by three binges, ileum-targeted colesevelam effectively reduced blood endotoxin levels. As shown in FIG. 9, measurement of the LPS/TLR4/CD14 inflammatory signaling pathway in the young rats revealed that oral administration of the ileum-targeted colesevelam reduced blood IL-1β levels, supporting the effectiveness of this treatment approach in reducing inflammation associated with alcoholic hepatitis.

### Example 13

The ileum-targeted colesevelam attenuates the systemic interleukin-6 levels of young rats with alcoholic hepatitis.

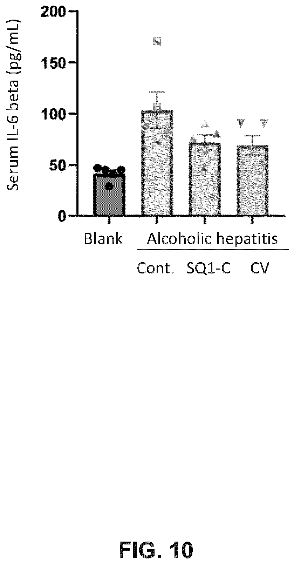

Interleukin-6 (IL-6) is a crucial inflammatory cytokine in liver disease progression. Serum IL-6 levels are significantly elevated in patients in conditions such as alcoholic or non-alcoholic cirrhosis and toxic hepatitis, compared to the control group. IL-6 can provoke alcoholic liver disease (ALD) by activating signal transducers and activators of transcription 3 (STAT3), which then promotes the expression of various hepatoprotective genes in hepatocytes. Clinical research has also identified serum IL-6 as a prognostic factor for alcoholic liver disease (ALD). Endotoxin can rapidly stimulate the expression of IL-6. Studies have shown that hepatocytes in response to endotoxin exposure can produce IL-6. Liver damage, however, can also impair the ability to clear up IL-6, resulting in persistent inflammatory pathogenesis. As shown in FIG. 10, by measuring the LPS/TLR4/CD14 inflammatory signaling pathway, oral administration of ileum-targeted colesevelam partially reduced the levels of the blood IL-6 inflammatory factor, supporting the effectiveness of this treatment approach in reducing inflammation associated with alcoholic hepatitis.

### Example 14

Assessment of fatty liver in the young rats subjected to alcoholic hepatitis.

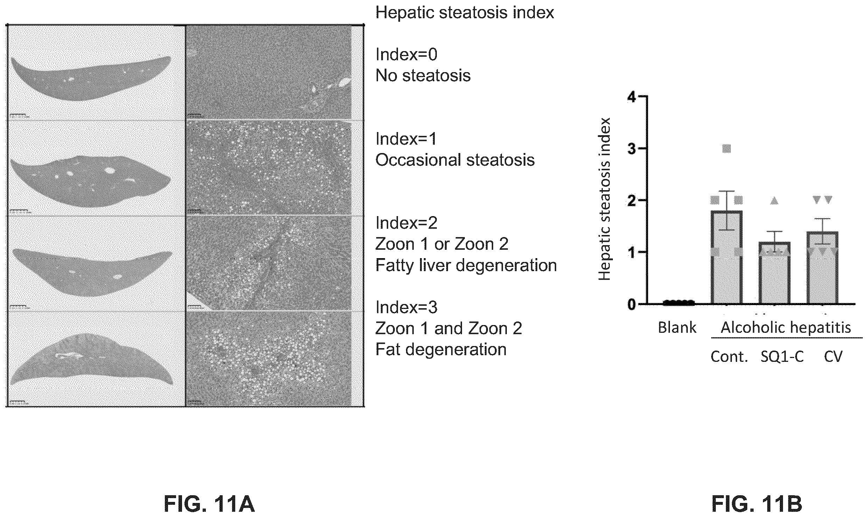

To quantitatively evaluate clinical samples and animal experimental data, the International Liver Disease Association classified fatty liver severity into mild, moderate, and severe stages. Common assessment indicators included the percentage of fat content in liver tissue or grading scores, with specific standards detailed as follows: F1 (mild fatty liver): showing the image of hepatic steatosis below 30% in area, generally regarded as having minimal impact on liver function. F2 (moderate fatty liver): steatosis level between 30% and 60% in area, where fat deposition may cause inflammatory and damage, moderately affecting liver function. F3 (severe fatty liver): steatosis content exceeding 60%, often accompanied by liver inflammation, fibrosis, and cellular damage, with significant impairement of liver function. The assessment of fatty liver was both qualitative and quantitative, usually determined by medical imaging techniques such as ultrasound, computed tomography, or magnetic resonance imaging, which estimated the degree of liver tissue fatty content. In the experiment, as shown in FIG. 7, liver tissues were subjected to hematoxylin and eosin (H&E) staining, and selected slides were presented according to the scoring standard, as shown in FIG. 11A.

### Example 15

The ileum-targeted formulation of colesevelam can improve alcoholic steatosis.

As depicted in FIG. 11B, the young rats subjected to Lieber-DeCarli alcohol feeding followed by three binges developed fatty liver. However, there was a significant individual variation within the group. In the alcoholic liver disease model group, one rat showed F3, two rats showed F2, and two rats showed F1. After oral administration of SQ1-C, two rats showed F2, while the other three showed F1. Importantly, the partial improvement of fatty liver observed in the rats treated with SQ1 was consistent with the reduction in systemic inflammation and the expression of hepatic inflammatory factors. The reduction further validated the hypothesis that SQ1-C could absorb intestinal endotoxins, reduce endotoxemia, and decrease systemic inflammation, thereby improve fatty liver.

### Example 16

Assessment method for liver inflammation in alcoholic hepatitis by experimental animal models.

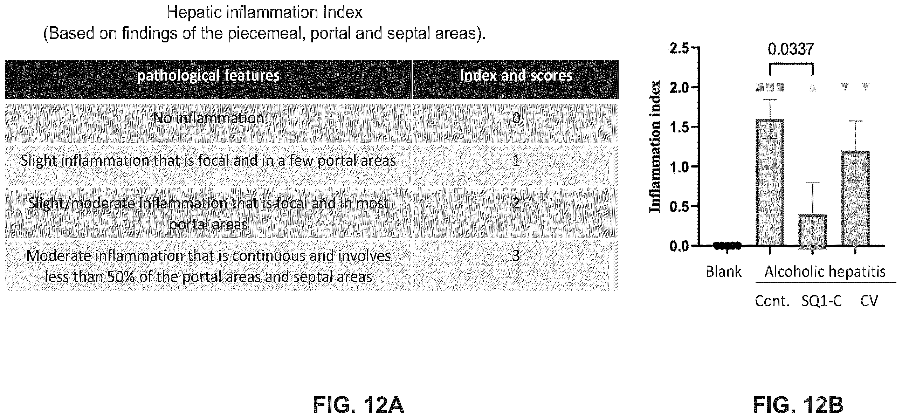

As shown in FIGS. 12A-12B, based on the rat liver sections and H&E staining results obtained from our experiments, we established the liver inflammation index as follows: Grade 0, fewer than 2 lesions in 10 fields of view at 200x magnification; Grade 1, exactly 2 lesions in 10 fields of view at 200x magnification; Grade 2, from 2 to 4 lesions in 10 fields of view at 200x magnification. Importantly, the ranking of liver inflammation index among groups is consistent with the levels of endotoxins and systemic inflammatory factors (IL-1), further illustrating the causal relationship of pathogenesis, that is, the key role of endotoxemia caused by intestinal bacterial toxins in the occurrence of liver disease.

### Example 17

The ileum-targeted colesevelam can suppress the mRNA expression of hepatic inflammatory factors in alcoholic liver disease.

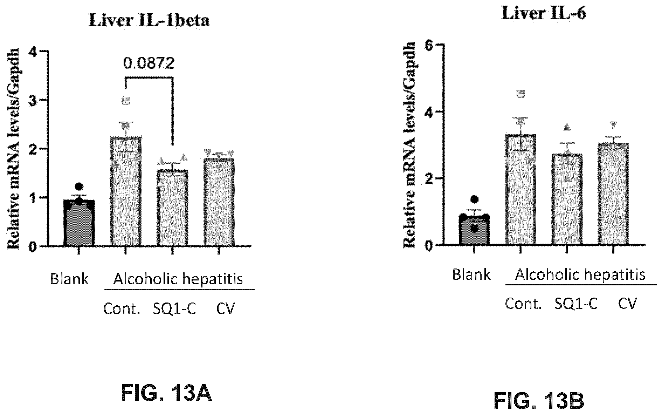

The expression of mRNA of two inflammatory factors in liver tissue was measured. As shown in FIGS. 13A-13B, six weeks of alcohol feeding coupled with three binge led to a 2-fold increase in the expression of the cytokine interleukin IL-1β and a 3-fold increase in IL-6 in liver tissue. Administration of the ileum-targeted colesevelam reduced the expression of these two inflammatory factors in liver tissue. Importantly, the expression of cytokine IL-1 is directly regulated by endotoxin/TLR4/CD14. Here, we show that its expression in the liver of alcoholic liver disease is completely consistent with the trend of blood endotoxin concentration, once again proving its causal pathological relationship. Moreover, we demonstrated that the SQ1 treatment can reduce the expression of hepatic inflammatory genes. This indicates that sequestration of intestinal endotoxin can be used to alleviate the expression of hepatic inflammatory genes.

### Example 18

Experimental design for the middle-aged rats under acute alcoholic hepatitis and treatment by ileum-targeted endotoxin sequestrants, SQ1C and SQ1T.

As depicted in FIG. 14, forty middle-aged rats (10-12 months old) were used in this experiment. Thirty of them were primed with alcohol in a Lieber-DeCarli diet (containing 5% alcohol in 36% calories) for 10 days. Then the animals were subjected to by one binge dose of 5 g/kg, 10 mL/kg body weight. The rats were terminated within 24 hours. During the experiment, two types of mini tablets, namely SQ1C and SQ1T, which are pH6.5-and-ileum targeted tablets with colesevelam and cholestyramine respectively, were orally administered at a dose of 91 mg/kg, with n=10 for each group.

### Example 19

Suppression of metabolic endotoxin levels in the rats under acute alcoholic hepatitis and treatment by ileum-targeted endotoxin sequestrants, SQ1-C and SQ1-T.

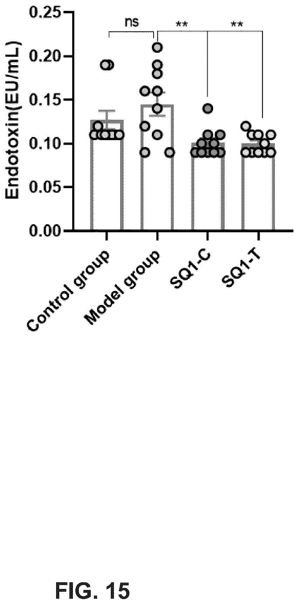

Alcohol consumption damages the intestinal barrier, increasing its permeability. This allows bacterial endotoxins (lipopolysaccharides, LPS) to leak from the gut into the bloodstream. Once in the bloodstream, LPS acts as a metabolic endotoxin. It binds to toll-like receptor 4 (TLR4) on immune cells such as Kupffer cells in the liver. This activates the immune system and triggers the production of pro-inflammatory cytokines like tumor necrosis factor-alpha (TNF-α), interleukin-1β (IL-1β), and interleukin-6 (IL-6). Large body of evidence including clinical study and animal work has demonstrated the impaired intestine in association with aging, which can further enhance transporting endotoxin into the liver and systemic circulation. The release of these cytokines leads to inflammation and oxidative stress in the liver. This can cause liver cell damage, steatosis (fat accumulation), inflammation (steatohepatitis), fibrosis (scarring), and eventually cirrhosis if the process continues. As shown in FIG. 15 for the method mentioned in FIG. 14, we measured the levels of metabolic endotoxin in the circulation of the middle aged rats with acute alcoholic hepatitis, being treated or not with endotoxin sequestrates in the ileum-targeted formulation. The middle-aged rats were primed with alcohol (5% in liquid diet, 36% calories from alcohol) for 10 days followed by one binge treatment (5g/kg body weight). This causes significant increase in plasma endotoxin levels, which is known as metabolic endotoxin. Notably, the pH6.5-designated and ileum-targeted copolymers, SQ1C and SQ1T can statistically suppress the plasma levels of endotoxin, measured by the LAL (Limulus Amebocyte Lysate) assay.

### Example 20

Systemic inflammation in acute alcoholic hepatitis can be suppressed by the pH6.5 designated and ileum-targeted endotoxin sequestrants, SQ1C and SQ1T.

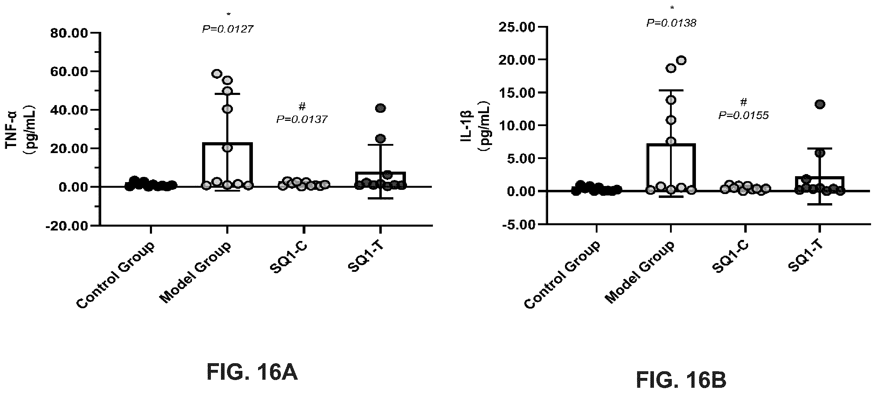

It is well documented that endotoxin can directly induce systemic inflammation marketed as elevation of interleukin-1beta and TNF-alpha in various metabolic disorders including alcoholic hepatitis. In alcoholic liver disease, IL-1β is an important mediator of inflammation. It is upregulated in response to alcohol-induced liver injury. IL-1β can be induced by bacterial endotoxins entering the bloodstream and activating the immune system. IL-1β is significantly associated with key clinical symptoms of acute alcoholic hepatitis such as fever, neutrophilia (increased number of neutrophils in the blood), and cachexia (wasting syndrome). TNF-alpha is also a key inflammatory cytokine that is involved in the inflammatory response in alcoholic diseases. Alcohol consumption can lead to an increase in TNF-alpha production. High levels of TNF-alpha are associated with the progression of alcoholic liver disease from steatosis (fat accumulation in the liver) to more severe forms such as steatohepatitis (inflammation and damage in the liver with fat accumulation), fibrosis (scarring of the liver), and cirrhosis (advanced scarring of the liver). As shown in FIGS. 16A-16B for the method mentioned in FIG. 14, the circulation levels of interleukin-1beta and TNF-alpha were promptly increased in the middle-aged rats in response to the acute alcoholic hepatitis. Conversely, the pH-sensitive and ileum-targeted colesevelam (SQ1-C) and cholestyramine (SQ1-T) are both able to suppress systemic inflammation in acute alcoholic hepatitis. First, this result is in agreement with reduction of plasma levels of endotoxin through the gut sequestration by two types of copolymers, targeted delivery by pH-sensitive and time-dependent formulation designated for ileal release. Second, the results showing attenuation of systemic inflammation serves as the basis of numerous therapeutic potentials. The cytokine levels in blood of the rats were measured by the ELISA assay.

### Example 21

The surged plasma levels of transaminases and liver injury in rats under acute alcoholic hepatitis are attenuated by oral administration of pH6.5-designated and ileum-targeted endotoxin sequestrants, SQ1-C and SQ1-T.

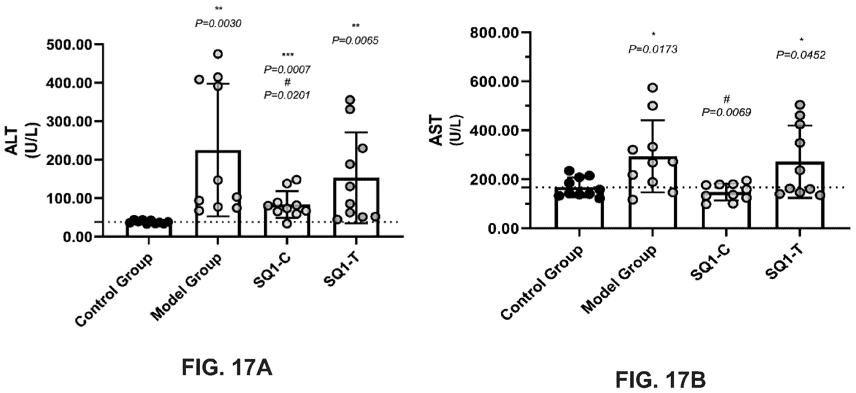

As shown in FIGS. 17A-17B, the pH-sensitive and ileum-targeted copolymers, SQ1C (colesevelam as API) and SQ1T (cholestyramine as API) are able to suppress the surged levels of transaminase and alleviate the liver functions and suppress liver injury in the middle-aged rats under acute alcoholic hepatitis. As illustrated in the figure, in middle-aged rats, alcohol priming combined with one binge treatment statistically leads to an elevation of serum levels of hepatic transaminases, alanine aminotransferase (ALT) and aspartate aminotransferase (AST). Significantly, SQ1-C and SQ1-T can ameliorate the liver injury, showing suppression of the transaminase levels.

### Example 22

Impairment of coagulation in acute alcoholic hepatitis is improved by ileum-targeted endotoxin sequestrants, SQ1-C and SQ1-T.

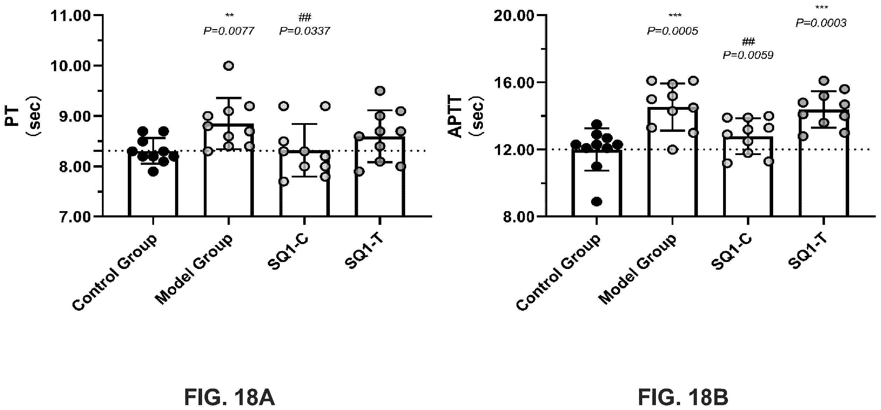

As shown in FIGS. 18A-18B and the experiment condition mentioned in FIG. 14, the coagulation system is defected in the rats under acute alcoholic hepatitis as indicated by the increased prothrombin time (PT) and activated partial thromboplastin time (APTT). Importantly, oral administration of the pH6.5-designated and ileum-targeted release of colesevelam as SQ1-C, and the corresponding cholestyramine as SQ1-T, can restore the coagulation homeostasis in the rats under acute alcoholic hepatitis.

### Example 23

Improvement of pancreatic injury in the rats under acute alcoholic hepatitis by oral administration of ileum-targeted endotoxin sequestrants, SQ1-C and SQ1-T.

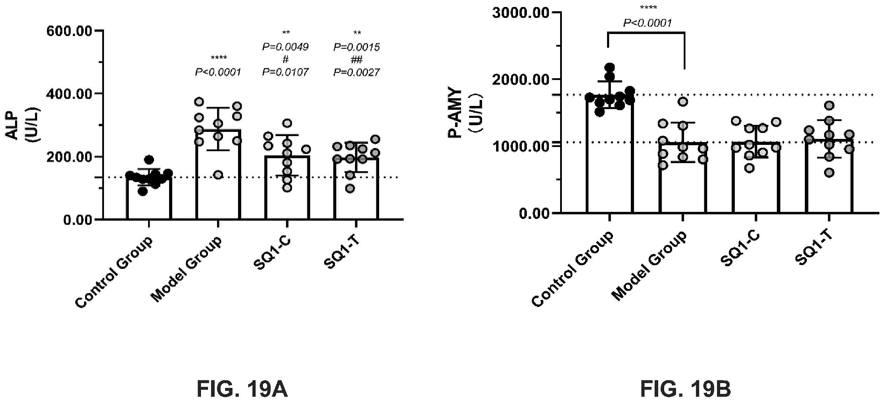

As shown in FIGS. 19A-19B, alcohol priming for 10 days followed by one binge led pancreatic injury, showing surged levels of plasma pancreatic markers, alpha-amylase (AMY) and alkaline phosphatase (ALP). Of note is that oral administration of SQ1-C and SQ1-T, two types of copolymeric API in the pH6.5-designated and ileum-targeted formulation can thoroughly suppress the pancreatic injury and reduce the pathological markers. Furthermore, the improvement of pancreatic function by the treatment of sequestration is in a tight association with reduction of metabolic endotoxin and systemic inflammation, suggesting a potential causality for the basis of the new treatment.

### Example 24

Hyperglycemia in alcoholic hepatitis is attenuated by oral administration of ileum-targeted endotoxin sequestrants, SQ1-C and SQ1-T

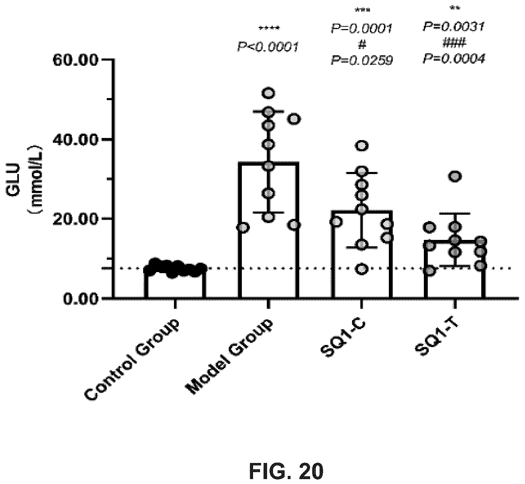

As shown in FIG. 20, alcohol drinking plus binge can significantly increase the plasma level of glucose, namely hyperglycemia in the middle-aged rats. Elevated glucose level or hyperglycemia is a common feature in alcoholic hepatitis. Remarkably, oral administration of pH6.5-designated and ileum-targeted colesevelam, SQ1-C, and cholestyramine, SQ1-T can attenuate the hyperglycemia in the rat model.

### Example 25

The GO (Gene Ontology) enrichment and KEGG enrichment analysis of the rats under acute alcoholic hepatitis and PASA treatment.

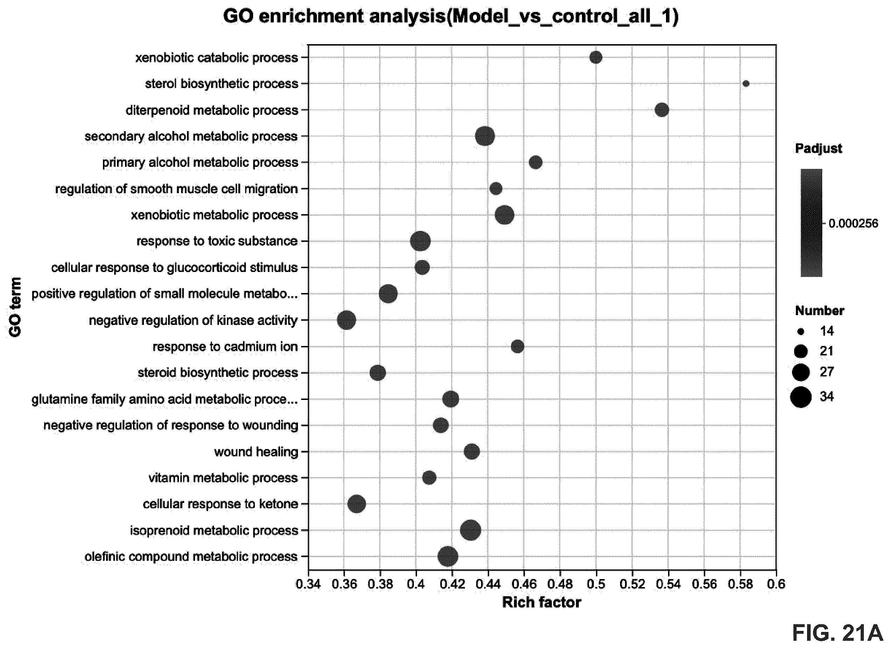

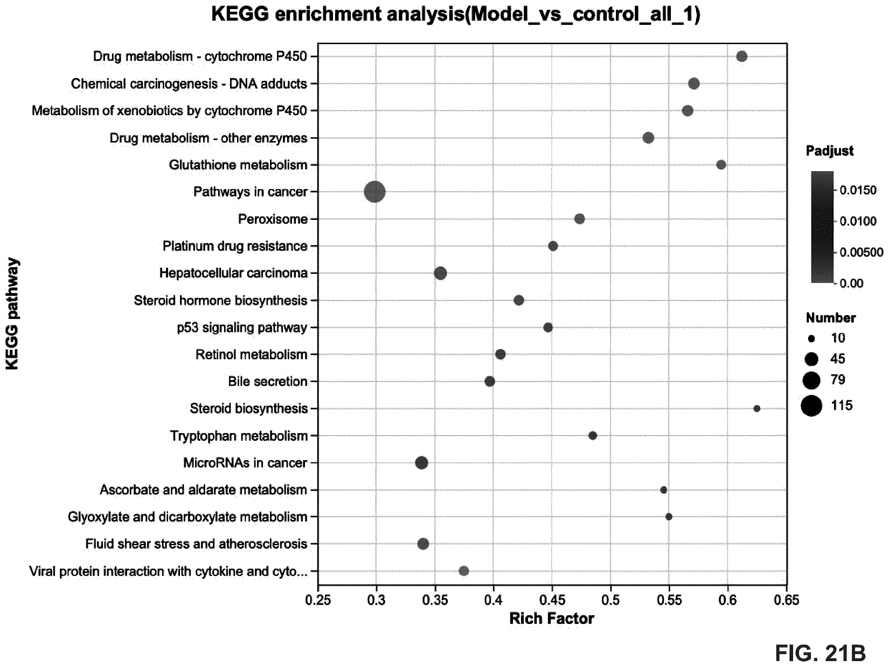

As shown in experiment design presented in FIG. 14, the liver tissue was processed for RNA extraction for RNA-seq analysis. The raw data from the RNA seq was further undergo the GO (Gene Ontology) enrichment analysis, which provides insights into the biological processes that are differentially regulated between the model group (the SD rats in acute alcoholic hepatitis) versus the control (FIGS. 21A-21B). The KEGG enrichment analysis results for "Model vs control all_1" provide insights into the differences between animals with alcohol binge (Model) and the control group in an animal model. The results showed that the following pathways are mobilized by alcohol drinking: xenobiotic catabolic process, sterol and steroid biosynthetic process, cellular responses to glucocorticoid stimulus and ketone.

### Example 26

The KEGG enrichment analysis for the hepatic gene expression by the rats under acute alcoholic hepatitis vs treatment with ileum-targeted endotoxin sequestrants, SQ1-C and SQ1T.

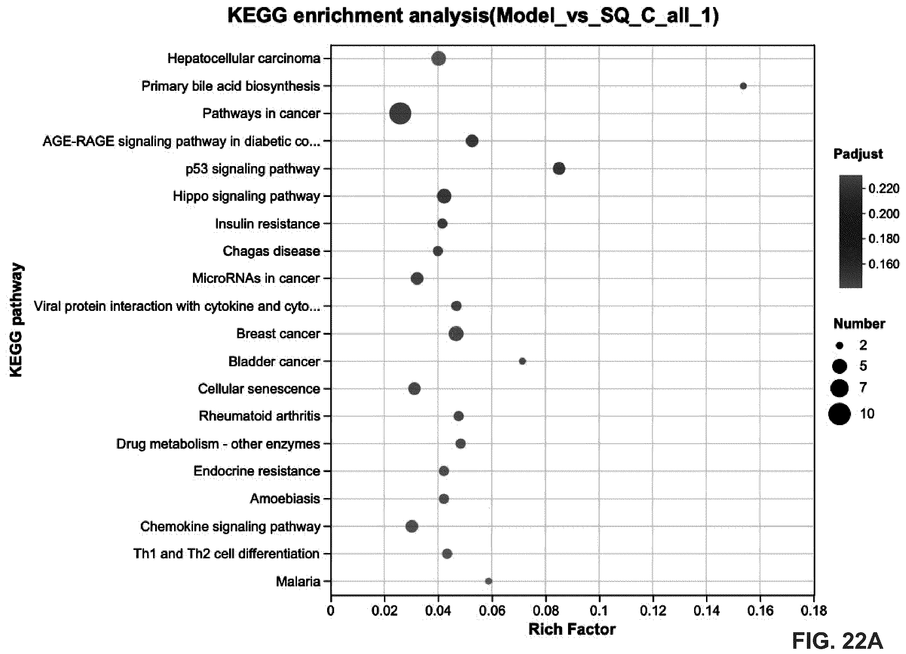

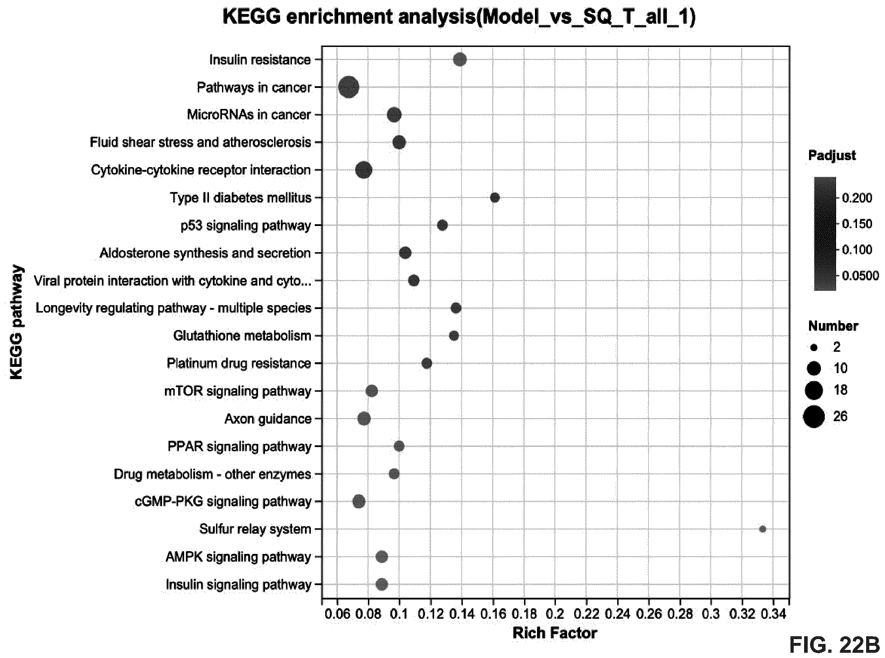

The KEGG enrichment analysis provides information about the biological pathways involved by the middle-aged rats under alcoholic hepatitis versus SQ1-C or SQ1-T sequestrating treatment. The experimental condition was mentioned in FIG. 14. This analysis suggests that alcohol binge affects multiple pathways related to diseases and biological processes (FIGS. 22A-22B). The endotoxin sequestration by ileum-targeted SQ1-C and SQ1-T may act on these pathways to counteract the negative effects of alcohol and potentially prevent or treat various diseases. The treatment with SQ1C and SQ1T appears to have an impact on the pathways related to hepatocellular carcinoma, diabetic complications, p53 and Hippo, insulin resistance, cellular senescence, rheumatoid arthritis, and drug metabolism, chemokine signaling, and Th1 versus Th2 cell differentiation.

### Example 27

Hepatic expression of TNF-alpha receptor and thrombospondin 1 (TSP-1), increased in the rats under acute alcoholic hepatitis, are suppressed through oral administration of ileum-targeted endotoxin sequestrant, SQ1-C fand SQ1-T.

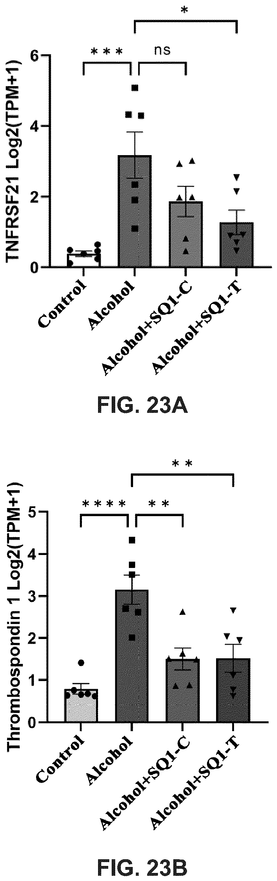

As described in FIG. 14, the middle-aged rats were primed with alcohol for ten days followed by a binge treatment, the gene expression in the liver tissues was analyzed by RNA-seq. As shown in FIGS. 23A-23B, hepatic expression of TNF-alpha receptor and thrombospondin 1 was evidently increased by alcohol treatment, in line with the endotoxin levels, the systemic inflammation, contributed by leukocytes in the body. Here we show liver tissue has intrinsic capacity to express TNF-alpha receptor, indicating hepatic source of inflammation. TSP-1 can promote fibrinolytic system, affect blood clot dissolution, and enhance systemic inflammation and disease severity in acute-on-chronic liver failure. It is known that LPS can induce the production of TSP-1. Strikingly, oral administration of ileum-targeted copolymeric sequestrants, SQ1-C and SQ1-T, can suppress hepatic expression of TNF-alpha (panel A) and thrombospondin 1 (panel B).

### Example 28

Hepatic fibrogenesis in the rats under alcoholic hepatitis is attenuated by oral administration of ileum-targeted sequestration of intestinal endotoxin.

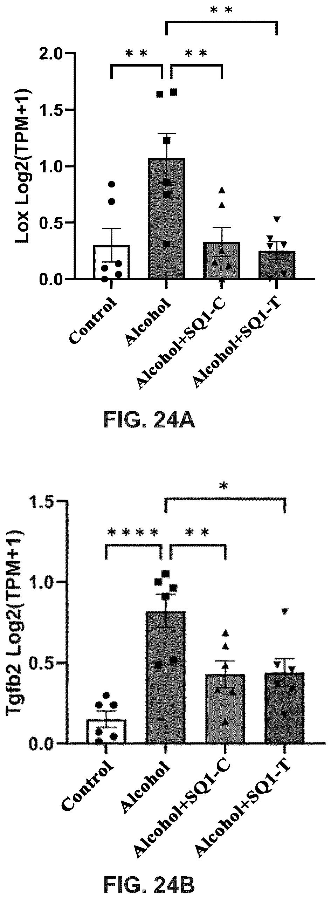

Lysine oxidase 2 is an enzyme belonging to the lysyl oxidase family. It plays a crucial role in the maturation and stabilization of the extracellular matrix (ECM). LOX2 mainly catalyzes the oxidative deamination of lysine residues in collagen and elastin, forming aldehyde groups. These aldehyde groups can further cross-link with other lysine residues or other molecules, thereby making the structures of collagen and elastin more stable. Likewise, TGF-β2 is a member of the transforming growth factor-β superfamily. It is a multifunctional cytokine that plays a crucial role in various biological processes such as cell growth, differentiation, apoptosis, and extracellular matrix (ECM) regulation. TGF-β2 stimulates HSCs to produce and secrete ECM components. The increased production of collagen, laminin, and fibronectin leads to the accumulation of ECM in the liver. This accumulation gradually changes the architecture of the liver, resulting in the replacement of normal liver parenchyma with fibrotic tissue. The cross-linking of ECM components is also enhanced under the influence of TGF-β2, making the ECM more rigid and less degradable. The experiment and treatment were described in FIG. 14. As shown in FIGS. 24A-24B, the rats under acute alcoholic hepatitis exhibited hepatic fibrogenesis and pathological progression, showing increased expression of hepatic lysine oxidase 2 (Lox 2, panel A) and transforming growth factor beta-2 (Tgfb2, panel B) in their mRNA levels. Compelling evidence ranging from clinical association and genetic experiment has demonstrated critical role of endotoxin signaling in liver fibrosis and activation of hepatic stellate cells.

## Claims

1. A method of treating a subject having a metabolic disorder, caused or promoted by pathogen-associated molecular patterns (PAMPs) derived from intestinal sources, the method comprising:
administering to the subject a drug that is targeted to deliver a sequestrant into distal region a small intestine for sequestration of PAMPs comprising endotoxins or CpG-DNA derived from gut microbes, wherein:
the sequestrant binds to the PAMPs to form a sequestrant-PAMP complex; and the sequestrant-PAMP complex is eliminated from digestive tract of the subject along with the rest of digestive contents.

2. The method of claim 1, wherein the sequestrant comprises high molecular co-polymers, which comprise three-dimensional framework of macromolecular hydrocarbon chains and having positively charged groups comprising amino groups.

3. The method of claim 1 or 2, wherein the sequestrant is formulated in the form of pellets, tablets or capsules for sequestration of the intestinal source of PAMPs; the pellets or tablet comprises a core granulate, an isolation layer as an option, and a pH-sensitive coating layer; while the core granulate comprises a copolymer as an active pharmaceutical ingredient (API).

4. The method of claim 3, wherein the pH-sensitive coating layer allows the sequestrant to be targeted in the ileum for therapeutic application.

5. The method of claim 3, wherein the copolymer is selected from the group consisting of colesevelam, cholestyramine, colestipol, and colestimide.

6. The method of claim 1, wherein the metabolic disorder is selected from the group consisting of alcoholic hepatitis, metabolic associated steatohepatitis (MASH), liver fibrosis and cirrhosis, liver failure, liver cancer, cardiovascular diseases (CVD), type-2 diabetes (T2D), and central obesity.

7. The method of claim 2, wherein the metabolic disorder is selected from the group consisting of alcoholic hepatitis, metabolic associated steatohepatitis (MASH), liver fibrosis and cirrhosis, liver failure, liver cancer, cardiovascular diseases (CVD), type-2 diabetes (T2D), and central obesity.

8. The method of claim 3, wherein the pH-sensitive coating layer comprises methyl methacrylate (MMA) and methacrylic acid (MAA).

9. The method of claim 8, wherein the pH-sensitive coating layer ensures that the sequestrant is released at a pH range of 6.5 to 7.5, allowing for targeted sequestration in the ileum.
